# EUROPEAN PATENT APPLICATION

(11) **EP 4 371 549 A1**
(43) Date of publication of application: **22.05.2024**
(21) Application number: 22841814.1
(22) Date of filing: 26.05.2022
(51) Int. Cl.: A61K 8/02, A45D 1/00, A45D 20/12, A61K 8/36, A61K 8/362, A61K 8/365, A61K 8/44, A61K 8/68, A61K 8/73, A61K 8/92, A61Q 1/00, A61Q 5/00

(54) **COSMETIC MATERIAL AND HAIRDRESSING APPLIANCE**

(30) Priority: 13.07.2021 JP 2021115572; 30.09.2021 JP 2021160358
(71) Applicant: Panasonic Intellectual Property Management Co., Ltd., Osaka-shi, Osaka 540-6207 (JP)
(72) Inventor: ISHIHARA Aya, Kadoma-shi, Osaka 571-0057 (JP); INOUE Hiroyuki, Kadoma-shi, Osaka 571-0057 (JP)
(74) Representative: Grünecker Patent- und Rechtsanwälte PartG mbB
(86) International application number: PCT/JP2022/021500
(87) International publication number: WO 2023/286467

(57) **Abstract**

A cosmetic material includes at least one organic substance, the at least one organic substance each being changeable to fine particles having an average particle size of less than or equal to 1 µm when being heated or pulverized, the cosmetic material being solid at normal temperature and not containing a liquid component, a cream-like component, or a gel-like component. A hairdressing appliance includes a cosmetic material holder that holds a cosmetic material, a heating unit that heats the cosmetic material held by the cosmetic material holder, and a jet unit that jets fine particles generated by heating by the heating unit to the outside.

## Description

### TECHNICAL FIELD

The present disclosure relates to a cosmetic material and a hairdressing appliance.

### BACKGROUND ART

Cosmetic materials for the skin or hair are directly applied to the skin or hair for the purpose of, for example, exhibiting a beauty effect on the skin or hair or maintaining or improving a physiological function. PTL 1 discloses, as a hair cosmetic material, that a specific organic acid, a specific organic solvent, and a specific dipeptide or tripeptide are contained to exhibit a hair modifying effect such as imparting setting properties to the hair and preventing dryness. PTL 2 discloses that stability at a high temperature is improved by containing hydrophobically modified polyether urethane, cellulose nanofibers, and water as a cosmetic material to be used by heating for obtaining an actual feeling of high effect.

### Citation List

### Patent Literatures

PTL 1: Unexamined Japanese Patent Publication No. 2011-157312
PTL 2: Unexamined Japanese Patent Publication No. 2020-142991

### SUMMARY OF THE INVENTION

Both of the cosmetic materials described in PTL 1 and PTL 2 are mixed with a base such as water or oil to facilitate application, and thus are easily oxidized and easily propagate bacteria. When an active ingredient is mixed with the base, the active ingredient easily permeates the skin and hair, but hardly stays on their surfaces, and it is difficult to act on the surfaces of the skin and hair. Further, when the cosmetic material is heated and used, it is difficult to maintain stability, and bacteria are more likely to propagate by heating.

The present disclosure has been made in view of such problems of the conventional technology. An object of the present disclosure is to provide a cosmetic material that is easy to handle, has high stability in storage, and can be attached to the skin or hair surface as fine particles, and a hairdressing appliance that can efficiently attach the cosmetic material to the skin or hair surface.

A cosmetic material according to a first aspect of the present invention includes at least one organic substance, the at least one organic substance each being changeable to fine particles having an average particle size of less than or equal to 1 µm when being heated or pulverized, and the cosmetic material is solid at normal temperature and does not contain liquid component, a cream-like component, or a gel-like component.

A hairdressing appliance according to a second aspect of the present disclosure includes a cosmetic material holder that holds a cosmetic material, the cosmetic material being solid at normal temperature, containing at least one organic substance, the at least one organic substance each being changeable to fine particles having an average particle size of less than or equal to 1 µm by heating, and the cosmetic material not containing a liquid component, a cream-like component, or a gel-like component, a heating unit that heats the cosmetic material held by the cosmetic material holder, and a jet unit that jets fine particles generated by heating the cosmetic material with the heating unit to an outside.

A hairdressing appliance according to a third aspect of the present invention includes a cosmetic material holder that holds a cosmetic material, the cosmetic material being solid at normal temperature, containing at least one organic substance, the at least one organic substance each being changeable to fine particles having an average particle size of less than or equal to 1 µm when being pulverized, and the cosmetic material not containing a liquid component, a cream-like component, or a gel-like component, a pulverizer that pulverizes the cosmetic material held by the cosmetic material holder, and a jet unit that jets fine particles generated by pulverizing the cosmetic material with the pulverizer to an outside.

A hairdressing appliance according to a fourth aspect of the present disclosure includes a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component, a heating unit that heats the cosmetic material held by the cosmetic material holder, and a jet unit that jets fine particles generated by heating the cosmetic material with the heating unit to an outside, in which the heating unit heats the cosmetic material by supplying hot air to the cosmetic material holder.

The present disclosure can provide a cosmetic material that is easy to handle, has high stability in storage, and can be attached to the skin or hair surface as fine particles, and a hairdressing appliance that can efficiently attach the cosmetic material to the skin or hair surface.

### BRIEF DESCRIPTION OF THE DRAWINGS

Fig. 1 is a conceptual diagram illustrating a configuration of a hairdressing appliance according to one exemplary embodiment of the present disclosure.
Fig. 2A is a schematic diagram illustrating a state in which fine particles of a cosmetic material according to one exemplary embodiment of the present disclosure remain on a skin surface.
Fig. 2B is a schematic diagram illustrating a state in which fine particles of the cosmetic material according to one exemplary embodiment of the present disclosure remain on a hair surface.
Fig. 3 is a graph illustrating a particle size distribution of a cosmetic material formed into fine particles according to one exemplary embodiment of the present disclosure.
Fig. 4 is a sectional view illustrating a hair dryer that is a hairdressing appliance according to one exemplary embodiment of the present disclosure.
Fig. 5 is a perspective view illustrating a straight iron that is a hairdressing appliance according to one exemplary embodiment of the present disclosure.
Fig. 6 is a perspective view illustrating a heat brush that is a hairdressing appliance according to one exemplary embodiment of the present disclosure.
Fig. 7 is a perspective view illustrating a facial treatment device that is a hairdressing appliance according to one exemplary embodiment of the present disclosure.
Fig. 8 is a perspective view illustrating a steamer that is a hairdressing appliance according to one exemplary embodiment of the present disclosure.
Fig. 9 is a schematic diagram illustrating an example of a cosmetic material conveyor in a hairdressing appliance according to one exemplary embodiment of the present disclosure.
Fig. 10 is a graph illustrating an atomization amount with respect to a duty ratio in intermittent control in a hairdressing appliance according to one exemplary embodiment of the present disclosure.
Fig. 11 is a graph illustrating a relationship between on/off (a) of intermittent control over time in the hairdressing appliance according to one exemplary embodiment of the present disclosure and atomization amount (B) with respect to the on/off.
Fig. 12A is a schematic diagram illustrating a state where a cosmetic material in a solid form and a heating unit are in contact with each other.
Fig. 12B is a schematic diagram illustrating a state where a cosmetic material in a solid form and a heating unit are not in contact with each other.
Fig. 13A is a schematic diagram illustrating a state where a cosmetic material in a solid form is irradiated with laser light by a laser light heater.
Fig. 13B is a schematic diagram illustrating a state where a cosmetic material in a solid form is not irradiated with laser light by a laser light heater.
Fig. 14 is a conceptual diagram illustrating a state where a cosmetic material and a heating auxiliary agent are heated by a laser light heater.
Fig. 15 is a graph illustrating a particle size distribution of a cosmetic material formed into fine particles when laser light irradiation is performed on the cosmetic material at high energy (solid line) and at low energy (broken line).
Fig. 16 is a conceptual diagram illustrating a configuration of a hairdressing appliance according to another exemplary embodiment of the present disclosure.
Fig. 17A is a perspective view illustrating the cosmetic material holder illustrated in Fig. 16.
Fig. 17B is an enlarged view of the inside of a circle indicated by A in Fig. 17A.
Fig. 17C is another enlarged view of the inside of the circle indicated by A in Fig. 17A.
Fig. 18 is a diagram for describing an example of a pulverizer that pulverizes a cosmetic material in a hairdressing appliance according to one exemplary embodiment of the present disclosure.
Fig. 19 is a diagram for describing another example of the pulverizer that pulverizes a cosmetic material in a hairdressing appliance according to one exemplary embodiment of the present disclosure.
Fig. 20 is a diagram for describing another example of the pulverizer that pulverizes a cosmetic material in a hairdressing appliance according to one exemplary embodiment of the present disclosure.
Fig. 21 is a diagram for describing a jet unit in a hairdressing appliance according to one exemplary embodiment of the present disclosure.

### DESCRIPTION OF EMBODIMENT

Hereinafter, exemplary embodiments will be described in detail with reference to the drawings. Unnecessarily detailed description may be omitted. For example, detailed descriptions of already well-known matters or redundant descriptions of substantially the same configuration may be omitted.

The accompanying drawings and the following description are only presented to help those skilled in the art fully understand the present disclosure and are not intended to limit the subject matters described in the claims.

### <Cosmetic material>

A cosmetic material according to one aspect of the present invention includes at least one organic substance, the at least one organic substance each being changeable to fine particles having an average particle size of less than or equal to 1 µm when being heated or pulverized, and the cosmetic material is solid at normal temperature and dese not contain a liquid component, a cream-like component, or a gel-like component. Since the cosmetic material of the present exemplary embodiment is solid at normal temperature, it is easy to handle, is stable even in long-term storage, is hardly oxidized, and is also difficult to propagate bacteria, and thus, the cosmetic material does not require a preservative. The cosmetic material can specifically act on the skin or hair surface by being directly applied to the skin or hair surface as solid fine particles without being mixed with a base such as a liquid component, a cream-like component, or a gel-like component. Further, a higher skin effect and hair effect can be obtained with a smaller amount than those of conventional cosmetic materials. In addition, it is possible to generate fine particles under a low-energy and atmospheric pressure environment.

The "normal temperature" is specifically 25°C. Examples of the liquid component include water, alcohol, and oil. The cream-like component and the gel-like component are components used as a base material of a cream-like cosmetic material or a gel-like cosmetic material.

The organic substance according to the present aspect can produce fine particles having an average particle size of less than or equal to 1 µm by heating or pulverization. The heating temperature at the time of producing fine particles varies depending on the melting point of the organic substance to be used, but is preferably 50°C to 250°C, and more preferably 80°C to 250°C. When the heating temperature is 50°C to 250°C, which is a temperature that can be achieved in a hairdressing appliance, fine particles are relatively easily generated. The average particle size of the fine particles to be produced is less than or equal to 1 µm, and preferably less than or equal to 300 nm. When the average particle size is less than or equal to 1 µm, the particles can be atomized and uniformly attached to the surface of the skin or hair, and a high effect can be obtained with a small amount. In addition, the fine particles do not permeate the skin or hair but remain on the surface, and exhibit a cosmetic effect. Examples of the cosmetic effect on the skin include an astringent effect, an effect of adjusting intercellular lipids of the horny layer to increase water retainability, an effect of enhancing softness, an effect of promoting adhesion and detachment of horny layer cells, and an effect of discharging an unnecessary horny layer. Examples of the effect to the hair include an effect of improving waviness and shine, moisture retention, and permeation of active ingredients (other ingredients).

The average particle size of the generated fine particles is a median diameter of an integrated value of 50% in a particle size distribution measured by a laser diffraction/scattering method.

In the present aspect, the organic substance preferably has a melting point or a sublimation point within the heating temperature range described above. This is because the organic substance easily generates fine particles having an average particle size of less than or equal to 1 µm by heating. The organic substance used in the present aspect generates fine particles having an average particle size of less than or equal to 1 µm when the organic substance reaches its melting point or sublimation point by heating, and such fine particles can be generated only by heating. That is, when the organic substance is formed into fine particles having an average particle size of less than or equal to 1 µm, treatments other than heating are not necessarily required. Since the cosmetic material of the present aspect is applied to human skin or hair, an organic substance that is harmless to the human body is used as the organic substance.

The organic substance is preferably an organic acid. The organic acid is preferably at least one selected from the group consisting of a saturated fatty acid, a dicarboxylic acid, a hydroxycarboxylic acid, and an amino acid. Examples of the saturated fatty acid include myristic acid (melting point: 53.9°C), palmitic acid (melting point: 63. 1°C), stearic acid (melting point: 69.6°C), arachidic acid (melting point: 75.6°C), behenic acid (melting point: 81.5°C), and lignoceric acid (melting point: 86°C). Examples of the dicarboxylic acid include fumaric acid (sublimation point: 200°C). Examples of the hydroxycarboxylic acid include malic acid (melting point: 287°C). Examples of the amino acid include serine (melting point: 228°C), cysteine (melting point: 216°C), glycine (melting point: 232°C to 236°C), lysine (melting point: 215°C), histidine (melting point: 272°C), and proline (melting point: 220°C to 222°C). Among these organic acids, malic acid and fumaric acid are preferable.

Alternatively, the organic substance is preferably a lipid or chitosan. Examples of the lipid include phospholipids, sphingolipids, glycolipids, cholesterol, ceramides, and cholesterol esters.

Examples of the organic substance may further include: proteins such as collagen, elastin, and keratin; various peptides; proteoglycans; various vitamins; enzymes such as lysozyme chloride, protease, and papain; nucleic acids such as DNA nucleic acids and ribonucleic acids; antioxidant components such as astaxanthin, lutein, and catechin; hormones such as isoflavone, dutasteride, and finasteride; lipids such as lauric acid, myristic acid, palmitic acid, glycosphingolipids, behenyl alcohol, stearyl alcohol, cholesterol, and hydrogenated lecithin; carbohydrates such as trehalose, dextran, dextrin, pullulan, cyclodextrin, and maltitol; urea; glycyrrhizic acid; and dipotassium glycyrrhizinate.

In the cosmetic material of the present aspect, two or more types of organic substances may be contained, in which the organic substances may have different melting points. Containing two or more types of organic substances having different melting points can make the timing at which the fine particles are generated different or can change the generation amount according to the heating temperature.

The cosmetic material of the present aspect is solid at normal temperature, but the specific shape may be a powder form or a solid form (lump, sheet, and the like).

### <Hairdressing appliance>

A hairdressing appliance according to another aspect of the present invention includes a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component. The hairdressing appliance further includes a heating unit that heats the cosmetic material held by the cosmetic material holder. The hairdressing appliance further includes a jet unit that jets fine particles generated by heating by the heating unit to the outside. That is, the hairdressing appliance of the present aspect is a device that uses the cosmetic material of the above-described aspect and applies the cosmetic material to the skin or hair of a person.

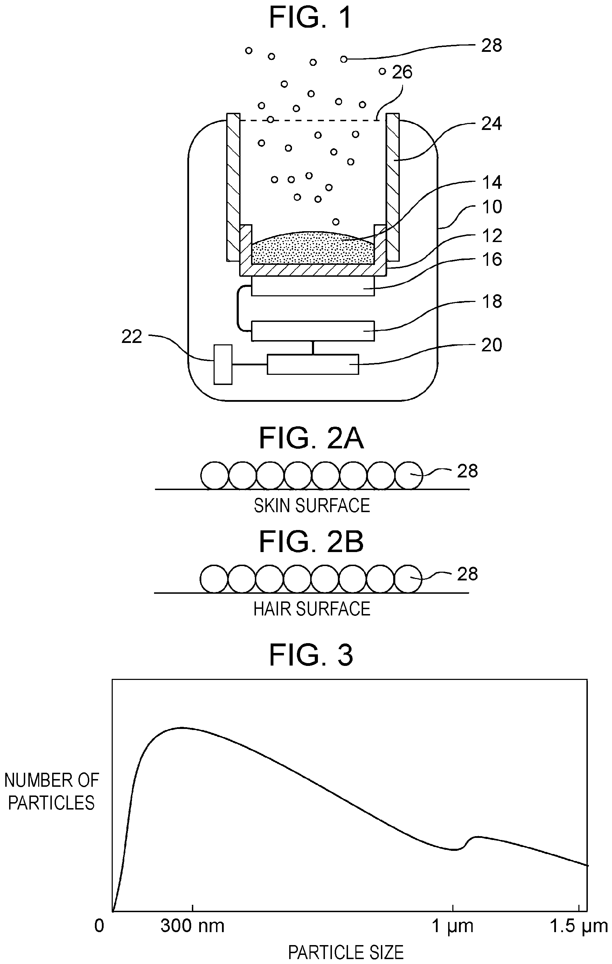

Fig. 1 is a conceptual diagram for describing a principle of a hairdressing appliance according to one exemplary embodiment of the present disclosure. Cosmetic material holder 12 in which cosmetic material 14 is accommodated and held is provided in housing 10. Heating unit 16 that heats cosmetic material 14 held by cosmetic material holder 12 through energization is provided below cosmetic material holder 12. Heating unit 16 is connected to controller 18, power supply (battery) 20, and switch 22 via an electric wire. Heating unit 16 is energized by turning on switch 22, and heating unit 16 generates heat. A heating state (heating temperature or the like) of heating unit 16 is controlled by controller 18.

Tubular part 24 extending upward is attached to cosmetic material holder 12. An uppermost end of tubular part 24 reaches an uppermost end of housing 10, and an uppermost part of tubular part 24 forms jet unit 26.

In the above configuration, when heating unit 16 is energized, cosmetic material 14 held by cosmetic material holder 12 is heated, and when cosmetic material 14 reaches the melting point, atomized fine particles 28 are generated. Atomized fine particles 28 generated rise in tubular part 24 because of the chimney phenomenon and are released from jet unit 26 to the outside. The released fine particles of the cosmetic material adhere to the skin or hair.

Fig. 2A is a schematic diagram illustrating a state where fine particles 28 of cosmetic material 14 remain on a skin surface. Fig. 2B is a schematic diagram illustrating a state where fine particles 28 of cosmetic material 14 remain on a hair surface. In the present exemplary embodiment, since the cosmetic material as fine particles 28 released by heating is fine particles, when the cosmetic material adheres to the skin surface or the hair surface, the cosmetic material remains on the skin surface or the hair surface as illustrated in Fig. 2A or 2B without permeating the skin surface or the hair surface. Thus, fine particles 28 of the cosmetic material continuously adhere to the surface of the skin or hair until the fine particles 28 come into contact with sebum or water, and thus, the effect of the cosmetic material is maintained.

In addition, fine particles 28 of the cosmetic material released as described above have a very small size (average particle size of less than or equal to 1 µm), can be uniformly attached to the skin or hair surface, and a high effect can be obtained with a small amount (less than 1/100 of that of a conventional cosmetic material using a base material). Fig. 3 illustrates an example of the particle size distribution of fine particles 28 of the cosmetic material of the present exemplary embodiment. According to Fig. 3, it can be seen that there is a peak of the particle size distribution in the vicinity of the particle size of 300 nm, which is extremely small.

The heating temperature by heating unit 16 is preferably set within a range of 70°C to 250°C, and the set temperature is preferably variable. In addition, to prevent denaturation of organic substances as the cosmetic material, it is preferable to perform heating in such a manner that the temperature does not become higher than necessary.

Heating unit 16 heats cosmetic material 14 to the above temperature, and it may be, specifically, an induction heater, a carbon heater, a positive temperature coefficient (PTC) heater, a coil heater, an infrared heater, or the like.

The hairdressing appliance of the present exemplary embodiment may further include an air blower that sends fine particles 28 generated by heating to the outside via jet unit 26 by blowing air. Generated fine particles 28 of the cosmetic material can fly farther in a certain direction by the air blower such as a fan. Thus, the effect is exhibited in a case where jet unit 26 of the hairdressing appliance and a part to which the cosmetic material is to be applied are separated from each other. In addition, by blowing air by the air blower, it is possible to uniformize the temperature of a part where fine particles are generated and reduce the density of the fine particles, and it is possible to prevent an increase in the particle size due to collision between the fine particles.

In the hairdressing appliance of the present exemplary embodiment, a configuration may be taken in which only a part of cosmetic material 14 is heated within a certain period of time. With this configuration, the frequency of recrystallization of the fine particles atomized in the air can be reduced, and coarsening of the fine particles can be prevented. By keeping the fine particle size small, the fine particle can be attached to the surface of the skin or hair in a smaller amount. Further, heat is not applied more than necessary to cosmetic material 14 at a part where fine particles are generated, and oxidation of the cosmetic material can be prevented. "Within a certain period of time" means that there is an upper limit to the time for heating only a part. That is, after only a part is heated and a certain time elapses, only another part can be heated.

In the hairdressing appliance of the present exemplary embodiment, it is preferable that a spatial concentration of fine particles 28 of the cosmetic material jetted from jet unit 26 is less than or equal to several tens mg/m³. By setting the spatial concentration of fine particles 28 of the cosmetic material to less than or equal to several tens mg/m³, an organic substance that emits odor can reduce its odor to such an extent that the odor is not noticeable.

Hereinafter, a hair dryer, a straight iron, a heat brush, a facial treatment device, and a steamer will be described as examples to which the hairdressing appliance of the present exemplary embodiment is applied.

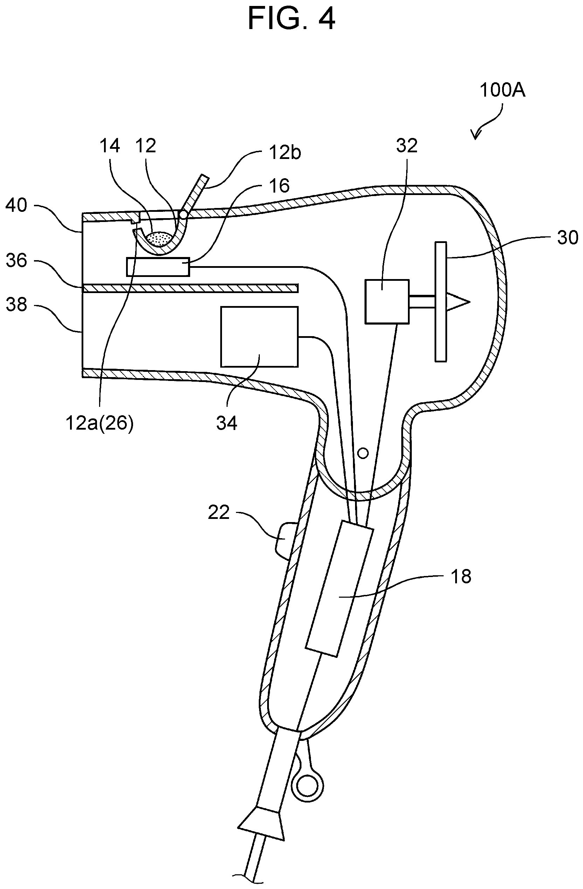

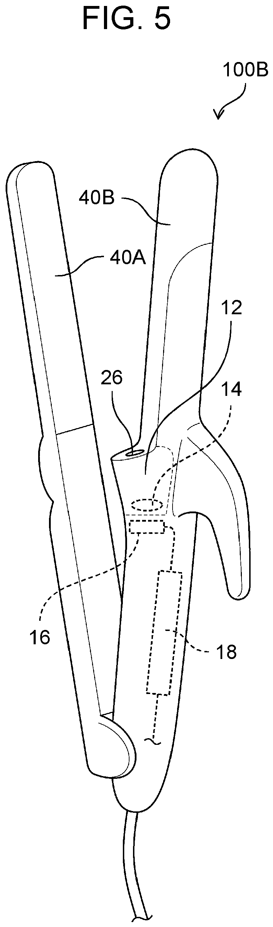

Fig. 4 illustrates hair dryer 100A. In Fig. 4, components that are substantially the same as the components illustrated in Fig. 1 are denoted by the same reference numerals. In Figs. 5 to 8 as well, components that are substantially the same as the components illustrated in Fig. 1 are denoted by the same reference numerals. Hair dryer 100A illustrated in Fig. 4 has a function of applying fine particles of the cosmetic material as described above to a typical hair dryer. Hair dryer 100A includes fan 30, motor 32 that drives fan 30, and heater 34 as functions of a typical dryer. That is, an air flow is generated by the rotation of fan 30, and the air flow is heated by heater 34 to become hot air. The hot air is sent out from blower port 38 positioned below partition wall 36. When heater 34 is in an off state, cold air is sent out. Motor 32 and heater 34 are electrically connected to controller 18, and controller 18 controls energization to each member according to the operation of switch 22.

The above is the function of a typical hair dryer. Hair dryer 100A further includes cosmetic material holder 12 that holds cosmetic material 14. Cosmetic material holder 12 includes a jet unit 12a and lid 12b that opens and closes vertically. Heating unit 16 is provided below cosmetic material holder 12, and heating unit 16 is electrically connected to controller 18. When heating unit 16 is in an on state, cosmetic material 14 in cosmetic material holder 12 is heated by heating unit 16 to form atomized fine particles, which are released from jet unit 12a. The released fine particles of the cosmetic material are released to the outside from blower port 40 positioned in the upper part of partition wall 36 by blowing air with fan 30. Thus, when the hair dryer is used, the fine particles of the cosmetic material are released together with hot air or cold air, and the fine particles of the cosmetic material can be applied to the hair.

In Fig. 4, cosmetic material holder 12 is provided on an upper part of a body of hair dryer 100A, but it may be provided at any position as long as fine particles of the cosmetic material are sent out by blowing air with fan 30.

Next, straight iron 100B illustrated in Fig. 5 will be described. Straight iron 100B is used to hold the hair between iron part 40A and iron part 40B that have generated heat, and straighten the hair with heat. Straight iron 100B includes cosmetic material holder 12 that holds cosmetic material 14. An upper part of cosmetic material holder 12 has jet unit 26 from which the cosmetic material heated and formed into fine particles is jetted. Heating unit 16 that heats cosmetic material 14 in cosmetic material holder 12 is provided below cosmetic material holder 12. Heating unit 16 is electrically connected to controller 18.

In the above configuration, when the hair is sandwiched between iron part 40A and iron part 40B to be straightened, the cosmetic material is atomized and jetted from jet unit 26 when cosmetic material 14 held in cosmetic material holder 12 is heated by heating unit 16. Thus, the fine particles of the cosmetic material can be applied to the hair while straight iron 100B is used.

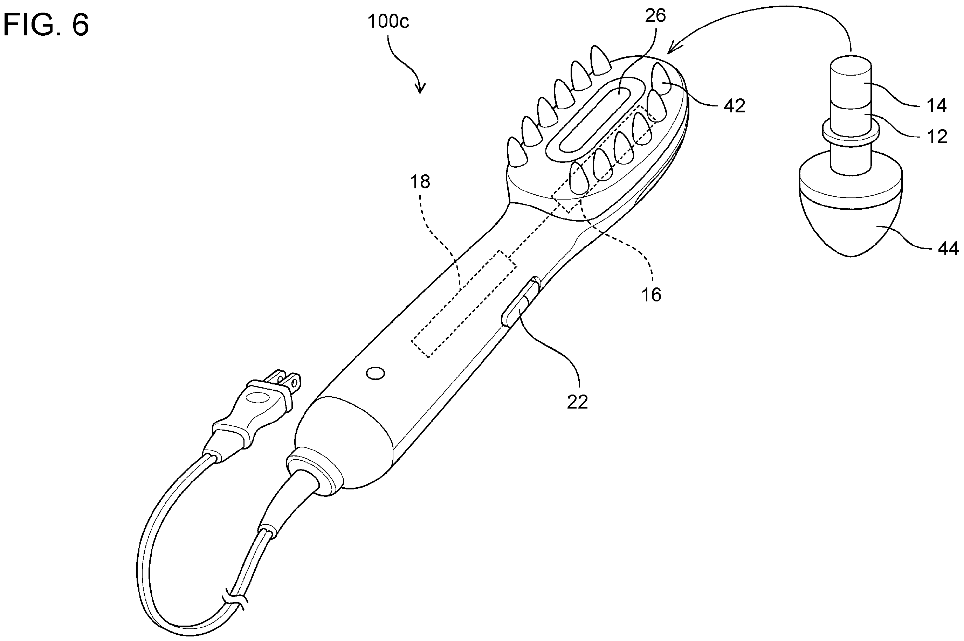

Next, heat brush 100C illustrated in Fig. 6 will be described. Heat brush 100C illustrated in Fig. 6 includes a large number (12 in Fig. 6) of brushes 42 that generate heat, and heats the hair to adjust and maintain a hairstyle. Heat brush 100C includes attachment 44 as a component to be attached to an upper end part (a part indicated by an arrow in Fig. 6), and attachment 44 includes cosmetic material holder 12 that holds cosmetic material 14. The body of heat brush 100C includes heating unit 16 positioned below cosmetic material holder 12 when attachment 44 is attached to the body of heat brush 100C. The body of the heat brush also includes jet unit 26 positioned above cosmetic material holder 12 when attachment 44 is attached to the body of heat brush 100C. Heating unit 16 is electrically connected to controller 18, and on/off of heating unit 16 is controlled by operating switch 22. When brush 42 of heat brush 100C is heated to adjust a hairstyle, cosmetic material 14 held by the cosmetic material holder 12 is heated by heating unit 16, and cosmetic material 14 becomes fine particles and is jetted from jet unit 26. Thus, the fine particles of the cosmetic material can be applied to the hair while heat brush 100C is used.

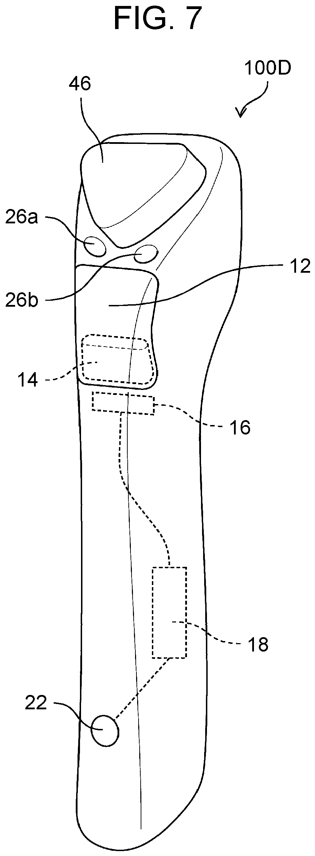

Next, facial treatment device 100D illustrated in Fig. 7 will be described. Facial treatment device 100D illustrated in Fig. 7 includes head 46 that heats or cools the skin of a face. Facial treatment device 100D includes cosmetic material holder 12 in which cosmetic material 14 is held, and jet units 26a, 26b through which the cosmetic material heated and formed into fine particles is jetted are provided in an upper part of cosmetic material holder 12. Heating unit 16 that heats cosmetic material 14 in cosmetic material holder 12 is provided below cosmetic material holder 12. Heating unit 16 is electrically connected to controller 18. Controller 18 controls energization to each member according to the operation of switch 22. When cosmetic material 14 held in cosmetic material holder 12 is heated by heating unit 16 at the time of performing a facial treatment using head 46 of facial treatment device 100D, the cosmetic material is formed into fine particles and jetted from jet units 26a, 26b. Thus, fine particles of the cosmetic material can be applied to the skin while facial treatment device 100D is used.

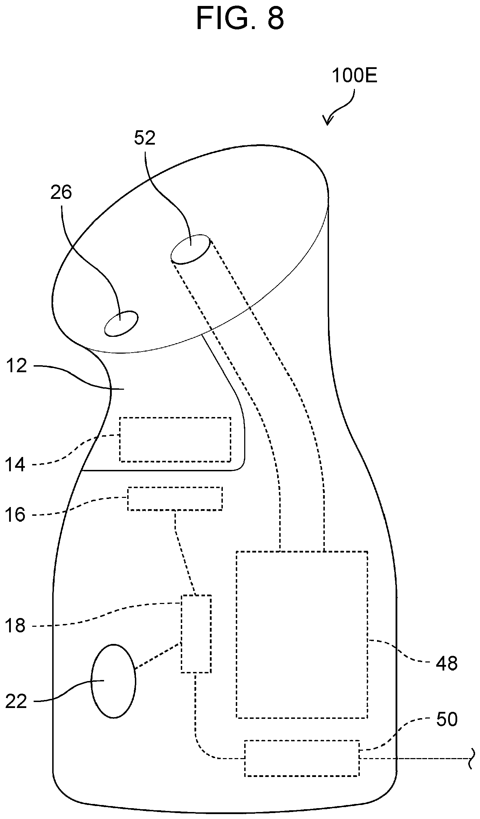

Next, steamer 100E illustrated in Fig. 8 will be described. Steamer 100E illustrated in Fig. 8 is a device that generates and discharges steam toward the skin or hair to perform skin care and hair care. As a steam generation mechanism, water tank 48 and heating unit 50 positioned below water tank 48 are provided. Water heated by heating unit 50 is discharged as steam from jet unit 52 to the outside. Steamer 100E includes cosmetic material holder 12 in which cosmetic material 14 is held, and in an upper part of cosmetic material holder 12, jet unit 26 through which the cosmetic material heated and formed into fine particles is jetted is provided. Heating unit 16 that heats cosmetic material 14 in cosmetic material holder 12 is provided below cosmetic material holder 12. Heating unit 16 is electrically connected to controller 18. Controller 18 controls energization to each member according to the operation of switch 22. When steam is generated and discharged by steamer 100E to perform skin care or hair care, the cosmetic material is formed into fine particles and jetted from jet unit 26 when cosmetic material 14 held by cosmetic material holder 12 is heated by heating unit 16. Thus, it is possible to apply fine particles of the cosmetic material to the skin and hair while discharging steam from steamer 100E.

When the cosmetic material held by the cosmetic material holder is in a powder form, the cosmetic material holder may further include a cosmetic material conveyor that conveys the cosmetic material in a powder form from the cosmetic material holder toward the heating unit, and a conveyor controller that controls the cosmetic material conveyor. In this case, the conveyor controller can perform control such that the cosmetic material is intermittently conveyed toward the heating unit. In each of the examples of Figs. 4 to 8, the heating unit is present in the vicinity of the cosmetic material holder. However, the cosmetic material holder and the heating unit may be separated from each other. In this case, it is preferable to provide a cosmetic material conveyor for conveying the cosmetic material to the vicinity of the heating unit.

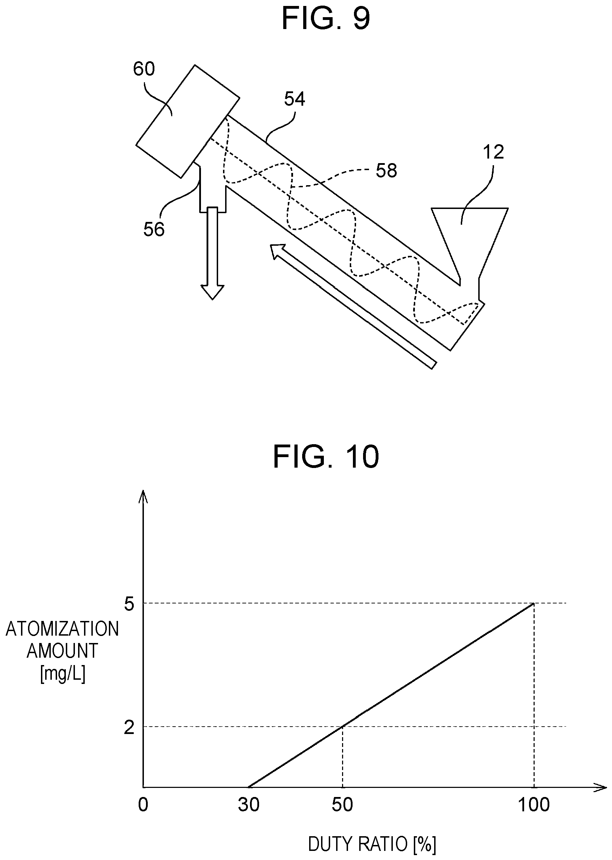

An example of the cosmetic material conveyor is illustrated in Fig. 9. The cosmetic material conveyor illustrated in Fig. 9 includes cylinder 54 having cosmetic material holder 12 that holds a cosmetic material in a powder form on one end side and carry-out unit 56 from which the cosmetic material in a powder form is carried out on the other end side. Screw 58 is provided inside cylinder 54, and motor 60 for driving is connected to an axis of rotation of screw 58. The powder cosmetic material held by cosmetic material holder 12 is conveyed upward by the rotation of screw 58. When the powder cosmetic material reaches the upper end part of cylinder 54, the powder cosmetic material falls downward from carry-out unit 56 and is carried out. Although not illustrated, a heating unit is provided below carry-out unit 56, and the cosmetic material in a powder form is heated by the heating unit.

The cosmetic material conveyor is controlled by a conveyor controller. The conveyor controller may be included in controller 18, for example. For example, the conveyor controller may control the conveyor in such a manner that the cosmetic material in a powder form is intermittently conveyed. When the cosmetic material in a powder form is controlled to be intermittently conveyed as described above, a certain amount of the cosmetic material is supplied to the heating unit at a time, and it is possible to reduce the time and effort for the user to charge the cosmetic material. In addition, the time until generation of fine particles is shortened, which contributes to power saving and energy saving. Further, deterioration of the cosmetic material due to repeated heating and cooling can be prevented.

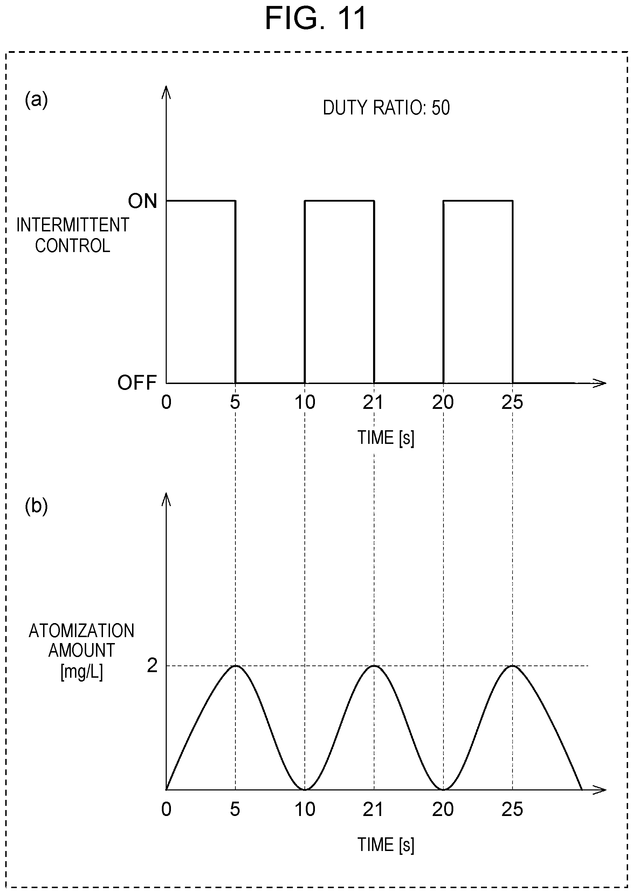

Fig. 10 illustrates a relationship between an atomization amount (fine particle amount) and a duty ratio in intermittent control for a cosmetic material to be formed into fine particles. Fig. 11 is a graph illustrating a relationship between on/off (a) of intermittent control over time and an atomization amount (b) with respect to the on/off. In Fig. 10, the atomization amount is 2 mg/L at a duty ratio of 50%. To realize such a duty ratio of 50%, on/off of conveyance may be controlled every 5 seconds as illustrated in (a) of Fig. 11. Such control results in a temporal change in the atomization amount as illustrated in (b) of Fig. 11.

When the cosmetic material is in a solid form, a movement mechanism that relatively moves the cosmetic material in a solid form and the heating unit in such a manner that the cosmetic material in a solid form and the heating unit are switched to either a contact state or a non-contact state, and a movement mechanism controller that controls the movement mechanism. The movement mechanism controller may be included in controller 18, for example. The movement mechanism controller can perform control such that the contact state and the non-contact state between the cosmetic material in a solid form and the heating unit are intermittently switched.

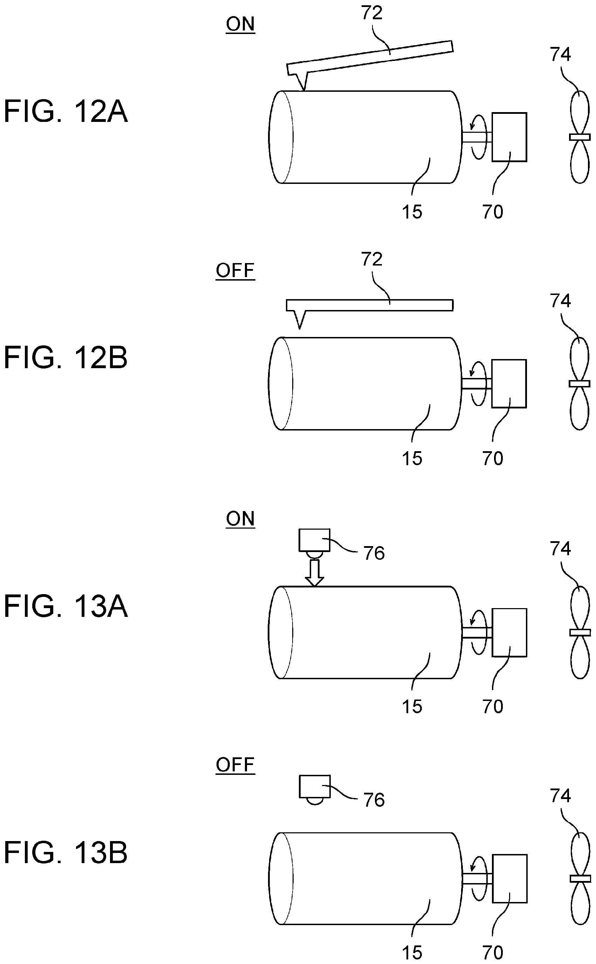

Figs. 12A and 12B illustrate a state in which control is performed such that the cosmetic material in a solid form and the heating unit are intermittently switched between the contact state and the non-contact state by relatively moving the heating unit with respect to cosmetic material 15 in a solid form having a circular cylinder shape. Heating unit 72 is moved by the movement mechanism between the contact state in which cosmetic material 15 in a solid form is in contact with heating unit 72 as illustrated in Fig. 12A and the non-contact state in which cosmetic material 15 in a solid form is not in contact with heating unit 72 as illustrated in Fig. 12B. That is, when cosmetic material 15 in a solid form is brought into contact with heating unit 72, cosmetic material 15 is heated to generate fine particles. In addition, motor 70 is connected to a central axis of cosmetic material 15 in a solid form having a circular cylinder shape, and cosmetic material 15 is rotated by driving of motor 70. Thus, the heated part of cosmetic material 15 is evenly heated over the periphery without concentration of heat on a part. Further, fan 74 is provided behind motor 70, and generated fine particles of the cosmetic material can be caused to fly away by blowing air with fan 74.

The movement of heating unit 72 as described above is controlled by a movement mechanism. For example, by intermittently performing the contact state and the non-contact state between cosmetic material 15 in a solid form and heating unit 72 at a constant timing, the contact frequency between the heating unit and the cosmetic material can be made constant, and the production amount of fine particles can be stabilized.

In the configuration illustrated in Figs. 12A and 12B, the heating unit is moved while cosmetic material 15 in a solid form is fixed, but the solid cosmetic material may be moved, or both the heating unit and the solid cosmetic material may be moved.

In the present exemplary embodiment, the heating unit may be a laser light heater that heats the cosmetic material by irradiation with laser light. In this case, it is preferable to have a configuration including a laser light controller that controls on/off or output of the laser light of the laser light heater, and a configuration in which a heating auxiliary agent to be heated by irradiation of the laser light of the laser light heater is held in the cosmetic material holder together with the cosmetic material. The laser light controller may be included in controller 18, for example. Then, the laser light controller performs control such that on/off of the irradiation of the cosmetic material and the heating auxiliary agent held by the cosmetic material holder with the laser light or the output of the laser light is intermittently performed. The laser light heater can perform pinpoint heating with power of several mW and can instantaneously heat the cosmetic material to generate fine particles.

An example of a mode in which a cosmetic material in a solid form is heated by a laser light heater will be described. Fig. 13A illustrates a state in which laser light heater 76 irradiates cosmetic material 15 in a solid form having a circular cylinder shape with laser light. Fig. 13A illustrates a case where laser light heater 76 is in an on state, and Fig. 13B illustrates a case where laser light heater 76 is in an off state. That is, cosmetic material 15 is heated to generate fine particles only when cosmetic material 15 in a solid form is irradiated with laser light by laser light heater 76. In addition, motor 70 is connected to a central axis of cosmetic material 15 in a solid form having a circular cylinder shape, and cosmetic material 15 is rotated by driving of motor 70. Thus, the heated part of cosmetic material 15 is evenly heated over the periphery without concentration of heat on a part. Further, fan 74 is provided behind motor 70, and generated fine particles of the cosmetic material can be caused to fly away by blowing air with fan 74.

The control of on/off or output of the laser light of the laser light heater 76 as described above is controlled by the laser light controller. For example, by intermittently turning on and off laser light at a constant timing, the frequency of irradiation of the cosmetic material with the laser light can be made constant, and the amount of fine particles generated can be stabilized.

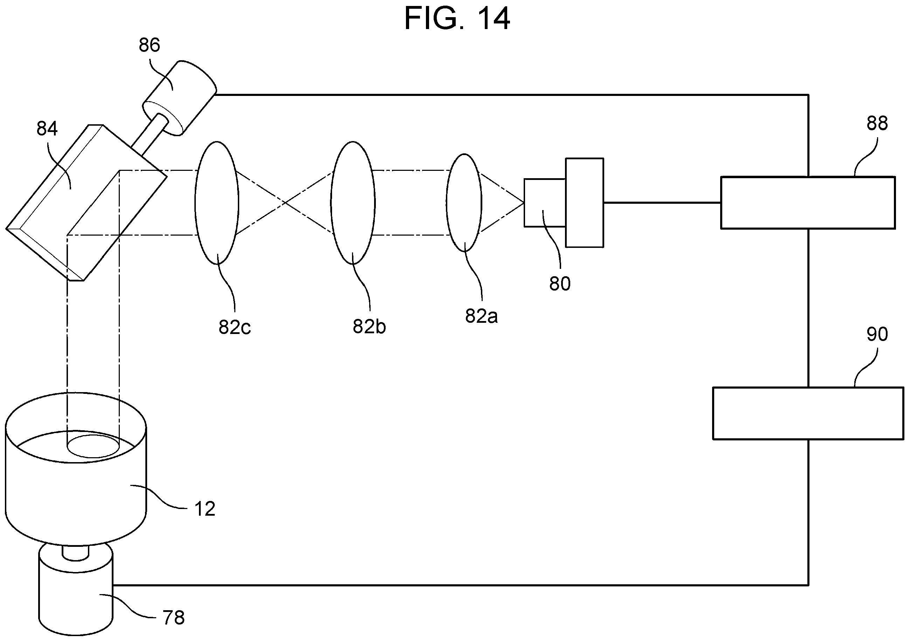

Fig. 14 is a diagram illustrating an example of the laser light heater. The laser light heater illustrated in Fig. 14 includes laser light source 80, lens 82a, lens 82b, lens 82c, galvanometer mirror 84, drive motor 86 that rotates galvanometer mirror 84, and light source controller 88 that controls laser light source 80. Laser light emitted from laser light source 80 passes through lens 82a, lens 82b, and lens 82c, is reflected by galvanometer mirror 84, and is incident on cosmetic material holder 12 positioned below galvanometer mirror 84. The cosmetic material holder 12 holds a mixture of the cosmetic material and a heating auxiliary agent to be heated by irradiation of laser light from the laser light heater. Cosmetic material holder 12 is rotated by motor 78.

When laser light emitted from laser light source 80 is incident on cosmetic material holder 12 and the heating auxiliary agent is irradiated with the laser light, the heating auxiliary agent generates heat. Then, when the cosmetic material is heated by heat generated by the heating auxiliary agent and reaches a melting point or higher, fine particles are generated. Thus, as in the heating by the heater described above, the cosmetic material formed into fine particles can be applied to the skin or hair by releasing the cosmetic material formed into fine particles to the outside.

The heating auxiliary agent to be heated by irradiation with laser light is a substance that absorbs laser light having a specific wavelength and generates heat, and is preferably selected according to the wavelength of the laser light to be irradiated. That is, it is possible to select a substance that generates heat according to the wavelength of laser light to be irradiated and use the selected substance as the heating auxiliary agent. Examples thereof include cerium oxide, carbon black, copper, iron, and nickel.

To efficiently heat the cosmetic material, the mixing ratio (mass basis) of the heating auxiliary agent to the cosmetic material is preferably 1/10 to 1/1.

Heating by the laser light heater is local, and only a part of the cosmetic material is heated within a certain period of time. By keeping the fine particle size small, the cosmetic material fine particles can be attached to the skin or hair surface in a smaller amount. In addition, heat is not applied to the cosmetic material at the site to be formed into fine particles, and oxidation can be prevented.

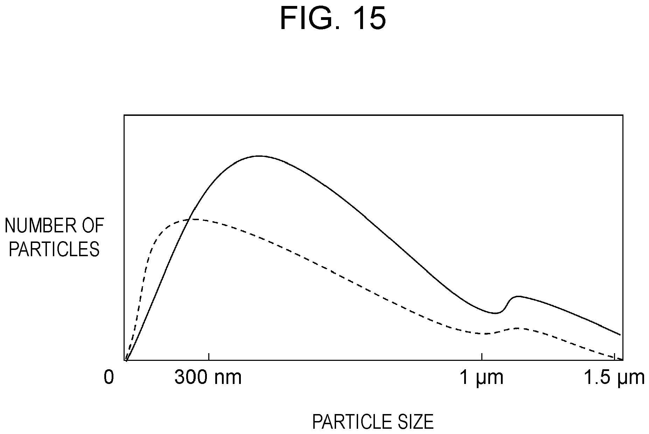

Fig. 15 illustrates the particle size distribution of the fine particles generated when the energy of laser light is high (solid line) and low (broken line). When the temperature (energy) is high, the particles are not easily cooled, and thus the particle size tends to increase. That is, to produce smaller fine particles, it is necessary to immediately lower the elevated temperature. Heating by laser light irradiation is suitable for producing smaller fine particles because the temperature drops as soon as the irradiation is stopped.

Further, in the present exemplary embodiment, the heating unit may be configured to heat the cosmetic material with hot air. In such a case, it is preferable to further include a temperature measurement unit that measures the temperature of the hot air and a hot air controller that controls at least one of the temperature of the hot air and the volume of the hot air based on the temperature measured by the temperature measurement unit. Then, the hot air controller controls the temperature of the hot air so that the temperature becomes equal to or higher than the melting point or sublimation point of the organic substance as a component of the cosmetic material, and thus, the produced fine particles can be jetted from the jet unit by the hot air to be applied to the skin or hair while fine particles are produced from the cosmetic material. The hot air delivered from the heating unit has a temperature equal to or higher than the melting point or sublimation point of the organic substance immediately after delivery and has a high temperature, but the temperature decreases due to energy consumption when the organic substance is formed into fine particles. Thus, high-temperature hot air is not directly applied to the skin or hair.

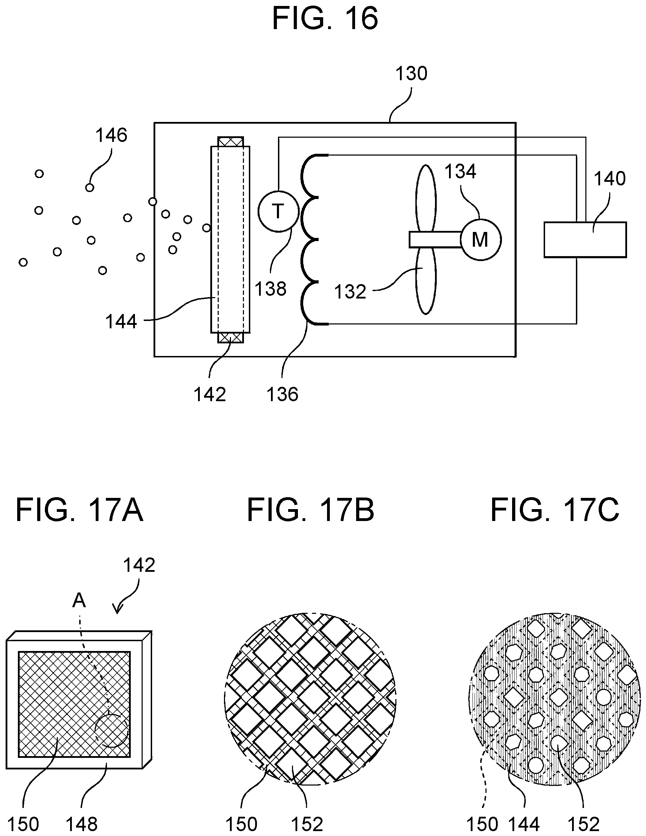

Fig. 16 is a conceptual diagram illustrating an example of a mode in which a cosmetic material is heated by hot air. Fan 132, motor 134 that drives fan 132, and heater 136 are provided in housing 130 of the hairdressing appliance. That is, an air flow is generated by the rotation of fan 132, the air flow is heated by heater 136 to become hot air, and the hot air reaches cosmetic material holder 142. Cosmetic material 144 is held in cosmetic material holder 142, and the organic substance as a component of cosmetic material 144 is heated by hot air, and when the temperature reaches the melting point of the organic substance, atomized fine particles 146 are generated.

Temperature measurement unit 138 that measures the temperature of hot air is provided on the downstream side of heater 136. Temperature measurement unit 138 and heater 136 are connected to hot air controller 140, and hot air controller 140 controls the voltage to be applied to heater 136 based on the temperature of the hot air measured by temperature measurement unit 138. Specifically, heater 136 is controlled in such a manner that the temperature of the hot air measured by temperature measurement unit 138 becomes equal to or higher than the melting point or the sublimation point of the organic substance as the cosmetic material.

Cosmetic material holder 142 is preferably formed of, for example, a mesh, a sintered body, or the like so that hot air can pass therethrough. When cosmetic material holder 142 is formed using a mesh, cosmetic material 144 is caused to attach to the mesh. At that time, it is preferable that cosmetic material 144 is attached in such a manner that the cosmetic material 144 does not completely block the opening of the mesh so that hot air can pass through the mesh. Figs. 17A to 17C illustrate an example in which cosmetic material holder 142 is formed using a mesh. Cosmetic material holder 142 illustrated in Fig. 17A has a form in which frame 148 supports mesh 150. Figs. 17B and 17C are enlarged views of the inside of a circle indicated by A in Fig. 17A. As shown in Fig. 17B, mesh 150 has a lot of openings 152. Fig. 17C illustrates a state in which mesh 150 holds cosmetic material 144. More specifically, cosmetic material 144 adheres to the front surface side and the back surface side of mesh 150, and a part thereof adheres to such an extent as to slightly close the opening. In Fig. 17C, even in a state where mesh 150 holds cosmetic material 144, opening 152 maintains a function as an opening, and hot air can pass therethrough.

Fig. 16 schematically illustrates a slightly exaggerated state of cosmetic material 144 being held by cosmetic material holder 142. Thus, the state of cosmetic material 144 held by cosmetic material holder 142 using mesh 150 illustrated in Figs. 17A to 17C is different from the state of cosmetic material 144 illustrated in Fig. 16.

Heater 136 may be any heater capable of turning the air flow generated by fan 132 into hot air having a temperature equal to or higher than the melting point or equal to or higher than the sublimation point. Specifically, the heater may be an induction heater, a carbon heater, a PTC heater, a coil heater, an infrared heater, or the like.

Hot air controller 140 can adjust the atomization amount of the cosmetic material by controlling the temperature of the hot air, the volume of the hot air, or the area of the hot air that hits the cosmetic material. The temperature control of the hot air can be performed by controlling the heater as described above. The air volume of hot air can be controlled by controlling a motor that drives a fan. Further, the area of hot air that hits the cosmetic material can be controlled, for example, by providing a variable opening that regulates the amount of air blown from the fan and controlling the variable opening.

The configuration described above is a configuration in which there is only one cosmetic material holder, but two or more cosmetic material holders may be provided, and the types of organic substances held in the respective cosmetic material holders may be different. In such a case, it is preferable to have a configuration including a heating unit controller that recognizes the types of the organic substances held in the two or more cosmetic material holders and controls the heating unit based on the recognized types of the organic substances. The heating unit controller may be included in controller 18, for example. Then, for example, although the melting points are different due to different types of organic substances, the heating unit controller sets a heating temperature suitable for the melting points of the respective organic substances and heats the organic substances, whereby the temperature can be raised in accordance with the cosmetic material, and oxidation and deterioration of the cosmetic material due to heat can be reduced. In addition, when two or more types of cosmetic materials are used, it is possible to change a timing at which fine particles are generated and a generation amount.

Examples of a method for recognizing the type of the organic substance held in the cosmetic material holder include a method of directly recognizing the type of the organic substance by measuring absorbance, density, and the like. Alternatively, the organic substance held in the cosmetic material holder may be determined in advance, and the heating unit controller may store the cosmetic material holder in which the determined organic substance is held, and control the cosmetic material holder based on the storage.

To perform optimum control according to the situation, the heating unit controller preferably has a configuration capable of individually setting and storing characteristics, a use state, and a use history of each cosmetic material.

In the above configuration, the cosmetic material is formed into fine particles by heating, but the cosmetic material may be formed into fine particles by pulverization. That is, the hairdressing appliance including the heating unit described above may be configured to include a pulverizer that pulverizes the cosmetic material held by the cosmetic material holder instead of the heating unit. By using a cosmetic material that can be formed into fine particles by pulverization, a substance that is easily oxidized by heat or a substance that is easily absorbed moisture can be formed into fine particles. In addition, the production amount of the fine particles can be stabilized by controlling the particle size.

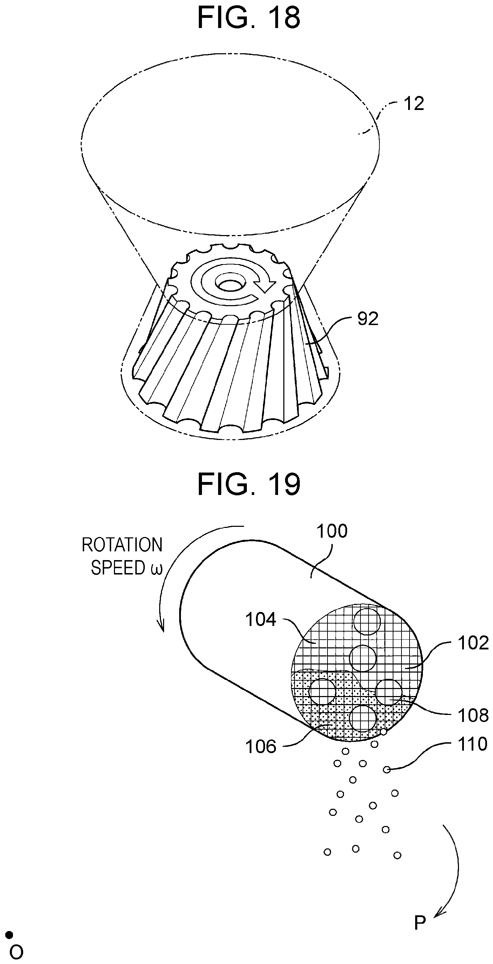

The pulverizer is not particularly limited as long as the cosmetic material is pulverized by pulverization. Specific examples of the pulverizer are illustrated in Figs. 18 to 20. The pulverizer illustrated in Fig. 18 includes rotary blade 92 inside a semi-conical part communicating with the lower side of cosmetic material holder 12. The rotary shaft of rotary blade 92 is connected to a motor (not illustrated). When the cosmetic material is charged into cosmetic material holder 12 and rotary blade 92 is rotated by driving of the motor, the cosmetic material is pulverized and formed into fine particles in the semi-conical extending part below cosmetic material holder 12.

The pulverizer illustrated in Fig. 19 includes bottomed cylindrical container 100 rotatably held inside the hairdressing appliance, and a cavity inside cylindrical container 100 forms cosmetic material holder 102. Filter 104 is attached to an open side edge of cylindrical container 100. Cosmetic material holder 102 has a plurality of pulverizing beads 108 having a diameter larger than the mesh of filter 104. Further, a drive motor (not illustrated) that rotates cylindrical container 100 about a cylindrical shaft as a rotation axis and a drive mechanism (not illustrated) that rotates cylindrical container 100 about the point O are provided inside the hairdressing appliance. That is, cylindrical container 100 can rotate (rotate) about the central axis and rotate (revolve) about the point O inside the hairdressing appliance.

Fig. 19 illustrates a state in which cosmetic material 106 is charged into cosmetic material holder 102. When cylindrical container 100 is rotated and revolved, the plurality of pulverizing beads 108 are concentrated on the outer peripheral part of cosmetic material holder 102 by centrifugal force, and cosmetic material 106 is pulverized by receiving stress due to collision or pressing by pulverizing beads 108 to generate fine particles 110. To obtain fine particles having an average particle size of less than or equal to 1 µm, the number of rotations (rotation speed) of cylindrical container 100, the rotation time, the material, the particle size, the number of pulverizing beads 108, and the like are appropriately set. Examples of the material of pulverizing beads 108 include zirconia, glass, alumina, zircon, and steel. The particle size of pulverizing beads 108 is preferably 3 mm to 8 mm. Pulverizing beads 108 may be 70% to 90% of the volume of cosmetic material holder 102.

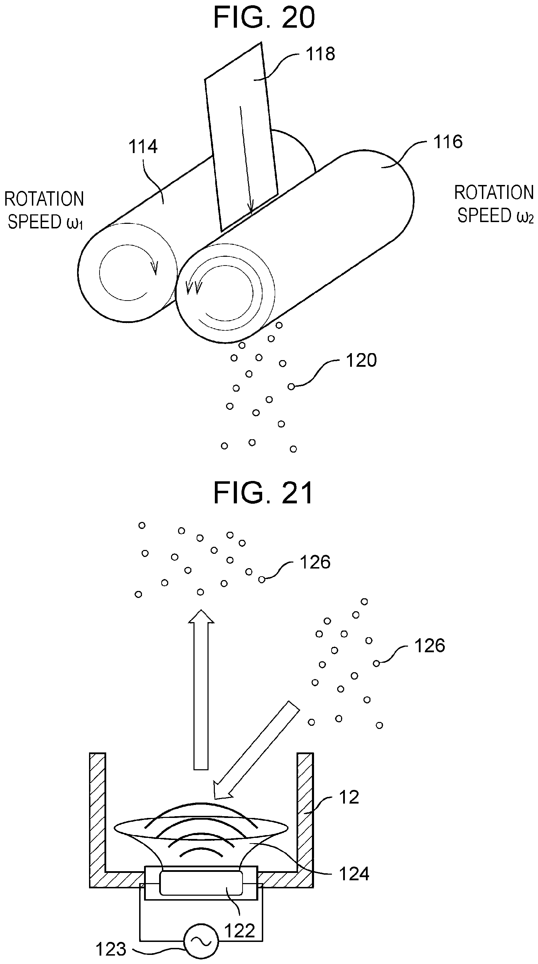

The pulverizer illustrated in Fig. 20 includes a pair of rollers 114 and 116 disposed in contact with each other. Rotation speed ω2 of roller 116 is set higher than rotation speed ω1 of roller 114. When the pair of rollers 114 and 116 rotate in such a state, cosmetic material 118 in a sheet form is charged between the pair of rollers 114 and 116. Then, cosmetic material 118 in a sheet form is pulverized by the compressive force and the shearing force from the pair of rollers 114 and 116, and fine particles 120 are generated. To obtain fine particles having an average particle size of less than or equal to 1 µm, rotation speed ω1 of roller 114 is preferably 150 rpm to 250 rpm, and rotation speed ω2 of the roller 116 is preferably 450 rpm to 550 rpm. In addition, it is preferable that the materials of rollers 114 and 116 have high hardness. For example, it is preferable to use zirconia, alumina, silicon nitride, or metal as the material of rollers 114 and 116.

Next, the jet unit that jets the fine particles generated by pulverizing the cosmetic material with the pulverizer to the outside is not particularly limited, and examples thereof include a jet unit using blowing air, a jet unit using vibration, and a jet unit using ultrasonic vibration. Of these, a jet unit using ultrasonic vibration will be described below.

Fig. 21 illustrates a jet unit that jets fine particles generated by pulverizing the cosmetic material with the pulverizer to the outside by using ultrasonic vibration. In Fig. 21, ultrasonic vibrator 122 and horn 124 are provided at the bottom of cosmetic material holder 12, and when a current is passed through ultrasonic vibrator 122 by power supply 123, ultrasonic vibration is generated from horn 124. Then, when fine particles 126 generated by pulverizing the cosmetic material with the pulverizer is served above horn 124, fine particles 126 are jetted by ultrasonic vibration.

Hereinafter, characteristic configurations of the cosmetic material and the hairdressing appliance according to the exemplary embodiment and effects obtained thereby will be described.
(1) A cosmetic material according to one aspect includes at least one organic substance, the at least one organic substance each being changeable to fine particles having an average particle size of less than or equal to 1 µm when being heated or pulverized, and the cosmetic material is solid at normal temperature and dese not contain a liquid component, a cream-like component, or a gel-like component. Since the cosmetic material is solid at normal temperature, it is easy to handle, is stable even in long-term storage, is hardly oxidized, and is also difficult to propagate bacteria, and thus a preservative is unnecessary. The cosmetic material can specifically act on the skin or hair surface by being directly applied to the skin or hair as solid fine particles without being mixed with a base such as a liquid component, a cream-like component, or a gel-like component. Thus, fine particles can be generated under a low energy environment in which a higher skin effect and hair effect can be obtained with a smaller amount than those of conventional cosmetic materials and under an atmospheric pressure environment.
(2) The organic substance may be an organic acid. Thus, various beauty effects can be obtained.
(3) The organic acid may be at least one selected from the group consisting of a saturated fatty acid, a dicarboxylic acid, a hydroxycarboxylic acid, and an amino acid. These are examples of the organic acid, and any organic acid that is harmless to the human body and exhibits a beauty effect can be used.
(4) The organic substance may be a lipid or chitosan. In addition to the organic acid, those typically used as cosmetic materials can also be used as long as they are solid at normal temperature and can produce fine particles having an average particle size of less than or equal to 1 µm by heating or pulverization.
(5) The cosmetic material may contain two or more types of organic substances that are the at least one organic substance, in which the organic substances may have different melting points. Containing two or more types of organic substances having different melting points can make the timing at which the fine particles are generated different or can change the generation amount according to the heating temperature. In this case, two or more types of organic substances may be contained in one cosmetic material holder, or one type of organic substance may be contained in each of two or more cosmetic material holders.
(6) A hairdressing appliance according to one aspect includes a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component. The hairdressing appliance further includes a heating unit that heats the cosmetic material held by the cosmetic material holder. The hairdressing appliance further includes a jet unit that jets fine particles generated by heating by the heating unit to the outside. Since the cosmetic material released by heating is fine particles, when the cosmetic material adheres to the skin surface or the hair surface, the cosmetic material remains on the skin surface or the hair surface without permeating the skin surface or the hair surface. Thus, fine particles of the cosmetic material continuously adhere to the surface of the skin or hair until the fine particles come into contact with sebum or water, and thus, the effect of the cosmetic material is maintained.
(7) The hairdressing appliance may further include an air blower that sends out the fine particles generated by heating to the outside through the jet unit by blowing air. Generated fine particles of the cosmetic material can fly farther in a certain direction by the air blower such as a fan.
(8) The heating unit may heat only a part of the cosmetic material within a certain period of time. The frequency of recrystallization of the fine particles atomized in the air can be reduced, and coarsening of the fine particles can be prevented.
(9) In the hairdressing appliance, the cosmetic material held by the cosmetic material holder may be in a powder form, and the hairdressing appliance may further include a cosmetic material conveyor that conveys the cosmetic material in a powder form from the cosmetic material holder toward the heating unit and a conveyor controller that controls the cosmetic material conveyor, The conveyor controller performs control such that the cosmetic material is intermittently conveyed toward the heating unit. When the cosmetic material holder and the heating unit are separated from each other, the cosmetic material can be heated by providing a cosmetic material conveyor for conveying the cosmetic material to the vicinity of the heating unit.
(10) In the hairdressing appliance, the cosmetic material held by the cosmetic material holder may be in a solid form, and the hairdressing appliance may further include a movement mechanism that relatively moves the cosmetic material in a solid form and the heating unit to switch the cosmetic material in a solid form and the heating unit to either a contact state or a non-contact state, and a movement mechanism controller that controls the movement mechanism. The movement mechanism controller performs control such that the contact state and the non-contact state between the cosmetic material in a solid form and the heating unit are intermittently switched. The heated part of the cosmetic material in a solid form is evenly heated over the periphery without concentration of heat on a part.
(11) In the hairdressing appliance, the heating unit may be a laser light heater that heats the cosmetic material by irradiation with laser light, and the hairdressing appliance may further include a laser light controller that controls at least one of on/off and output of the laser light of the laser light heater. In addition, the cosmetic material holder holds a heating auxiliary agent to be heated by irradiation of laser light from the laser light heater in addition to the cosmetic material. Then, the laser light controller may perform control such that at least one of the on/off of the irradiation of the cosmetic material and the heating auxiliary agent held by the cosmetic material holder with the laser light and the output of the laser light is intermittently performed. The laser light heater can perform pinpoint heating with power of several mW and can instantaneously heat the cosmetic material to generate fine particles.
(12) In the hairdressing appliance, the cosmetic material holder may include two or more cosmetic material holders, types of organic substances respectively held by the two or more cosmetic material holders may be different from each other, and the hairdressing appliance may further include a heating unit controller that recognizes the types of the organic substances respectively held in the two or more cosmetic material holder and controls the heating unit based on the types of the organic substances that are recognized.
(13) A hairdressing appliance according to an aspect includes a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component. The hairdressing appliance further includes a pulverizer that pulverizes the cosmetic material held by the cosmetic material holder. The hairdressing appliance further includes a jet unit that jets fine particles generated by pulverization of the cosmetic material by the pulverizer to the outside. The heating unit controller sets a heating temperature suitable for the melting points of the respective organic substances and heats the organic substances, whereby the temperature can be raised in accordance with the cosmetic material, and oxidation and deterioration of the cosmetic material due to heat can be reduced. In addition, when two or more types of cosmetic materials are used, it is possible to change a timing at which fine particles are generated and a generation amount.
(14) A hairdressing appliance according to an aspect includes a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component. The hairdressing appliance further includes a heating unit that heats the cosmetic material held by the cosmetic material holder. The hairdressing appliance further includes a jet unit that jets fine particles generated by heating by the heating unit to the outside. Then, the heating unit supplies hot air to the cosmetic material holder to heat the cosmetic material. Since the cosmetic material is heated with hot air to be formed into fine particles, an appropriate amount can be applied to the skin or hair at a desired timing.
(15) The hairdressing appliance may further include a temperature measurement unit that measures a temperature of the hot air, and a hot air controller that controls at least one of the temperature of the hot air and a volume of the hot air based on the temperature measured by the temperature measurement unit. By controlling the temperature of the hot air so as to be equal to or higher than the melting point or sublimation point of the organic substance and controlling the air volume of the hot air, the fine particle generation amount can be adjusted with a simple structure.
(16) The heating unit may be an induction heater, a carbon heater, a positive temperature coefficient (PTC) heater, a coil heater, or an infrared heater. However, the heating unit only needs to be able to heat the cosmetic material to a temperature equal to or higher than the melting point or the sublimation point with hot air, and the above-described heating unit is merely an example.
(17) The jet unit may jet fine particles generated by heating by the heating unit to the outside by air blowing, vibration, or ultrasonic vibration.
(18) The hairdressing appliance may further include a heating unit controller that adjusts the atomization amount of the cosmetic material by controlling the temperature of the hot air, the volume of the hot air, or the area of the hot air that hits the cosmetic material. It is possible to easily adjust the atomization amount of the cosmetic material by controlling the temperature, the air volume, and the area to which the cosmetic material is applied with respect to the hot air.

Next, in the present disclosure, an aspect equivalent to the above-described aspect but expressed in another expression or another aspect will be described.

(a) An organic acid fine particle forming application apparatus including: a fine particle formation unit that forms a solid organic acid into fine particles by heating; and a fine particle application unit that applies the organic acid formed into fine particles by the fine particle formation unit to a skin or hair.

In the mode illustrated in Fig. 1, the fine particle formation unit corresponds to the periphery of cosmetic material holder 12 and heating unit 16, and has a function of forming a solid organic acid into fine particles by heating. In the mode illustrated in Fig. 1, the fine particle application unit corresponds to the part from tubular part 24 to jet unit 26.

(b) The organic acid fine particle forming application apparatus according to (a), in which the organic acid includes a plurality of organic acids having different melting points, and the fine particle application unit sequentially applies the organic acids having different melting points.

When a plurality of organic acids having different melting points are used as the organic acid and the heating temperature is gradually increased, the organic acid having reached the melting point is sequentially formed into fine particles, and thus, organic acids having different melting points can be sequentially applied.

(c) A hairdressing appliance including: a fine particle formation unit that forms a solid organic acid into fine particles by heating; and a fine particle application unit that applies the organic acid formed into fine particles by the fine particle formation unit to a skin or hair.

As in the aspect of (a), in the mode illustrated in Fig. 1, the fine particle formation unit corresponds to the periphery of cosmetic material holder 12 and heating unit 16, and has a function of forming a solid organic acid into fine particles by heating. In the mode illustrated in Fig. 1, the fine particle application unit corresponds to the part from tubular part 24 to jet unit 26.

(d) The hairdressing appliance according to (c), in which the organic acid includes a plurality of organic acids having different melting points, and the fine particle application unit sequentially applies the organic acids having different melting points.

As in the aspect of (b), when a plurality of organic acids having different melting points are used as the organic acid and the heating temperature is gradually increased, the organic acid having reached the melting point is sequentially formed into fine particles, and thus, organic acids having different melting points can be sequentially applied.

(e) A cosmetic material storage container in which an organic acid that is solid at normal temperature is heated and stored in a state where the organic acid can be formed into fine particles.

As described above, the organic acid can be formed into fine particles by heating, but in the cosmetic material storage container of the present aspect, the organic acid is stored in such a state. For example, the organic acid is stored in a powder form.

(f) The organic acid fine particle forming application apparatus according to (a) or (b), further including an organic acid pulverization unit that pulverizes a solid organic acid.

Since the solid organic acid can be formed into fine particles by pulverization, the solid organic acid can be formed into fine particles by including the organic acid pulverization unit.

(g) The hairdressing appliance according to (c) or (d), further including an organic acid pulverization unit that pulverizes a solid organic acid.

As in the aspect of (f), by including the organic acid pulverization unit, the solid organic acid can be formed into fine particles.

(h) The organic acid fine particle forming application apparatus according to (a) or (b), in which laser light is used in heating with the fine particle formation unit.

By using laser light as a heating method in the fine particle formation unit, there is an advantage that the organic acid can be instantaneously heated and formed into fine particles as described above.

(i) The hairdressing appliance according to (c) or (d), in which laser light is used in heating with the fine particle formation unit.

As in the aspect of (h), by using laser light as a heating method in the fine particle formation unit, there is an advantage that the organic acid can be instantaneously heated and formed into fine particles as described above.

(j) The organic acid fine particle forming application apparatus according to (a) or (b), in which the fine particle application unit includes a spatial concentration controller that controls a spatial concentration of fine particles at the time of application.

By controlling the spatial concentration of fine particles at the time of application and increasing the spatial concentration, the effect of the organic acid as fine particles is improved. In addition, when the spatial concentration is lowered, an organic acid that emits odor can reduce its odor to such an extent that the odor is not noticeable.

(k) The hairdressing appliance according to (c) or (d), in which the fine particle application unit includes a spatial concentration controller that controls a spatial concentration of fine particles at the time of application.

As in the aspect of (j), by controlling the spatial concentration of fine particles at the time of application and increasing the spatial concentration, the effect of the organic acid as fine particles is improved. In addition, when the spatial concentration is lowered, an organic acid that emits odor can reduce its odor to such an extent that the odor is not noticeable.

(l) The organic acid fine particle forming application apparatus according to (a) or (b) provided with an organic acid automatic supply unit that automatically supplies a solid organic acid to be formed into fine particles in the fine particle formation unit from an organic acid storage unit storing the organic acid to the fine particle formation unit.

The organic acid automatic supply unit may have a function of automatically supplying the organic acid in the organic acid storage unit to the fine particle formation unit, and examples thereof include the cosmetic material conveyor as illustrated in Fig. 9.

(m) The hairdressing appliance according to (c) or (d) provided with an organic acid automatic supply unit that automatically supplies a solid organic acid to be formed into fine particles in the fine particle formation unit from an organic acid storage unit storing the organic acid to the fine particle formation unit.

As in (I), the organic acid automatic supply unit may have a function of automatically supplying the organic acid in the organic acid storage unit to the fine particle formation unit, and examples thereof include the cosmetic material conveyor as illustrated in Fig. 9.

(n) The organic acid fine particle forming application apparatus according to (l), in which an operation at the time of automatic supply of the organic acid automatic supply unit is an intermittent operation.

By intermittently operating the supply operation of the organic acid to the fine particle formation unit with the organic acid automatic supply unit, the organic acid is supplied to the fine particle formation unit by a certain amount. Thus, it is possible to reduce the time and effort for the user to charge the organic acid. In addition, the time until generation of fine particles is shortened, which contributes to power saving and energy saving. Further, deterioration of the organic acid due to repeated heating and cooling can be prevented.

(o) The hairdressing appliance according to (m), in which an operation at the time of automatic supply of the organic acid automatic supply unit is an intermittent operation.

As in (n), by intermittently operating the supply operation of the organic acid to the fine particle formation unit with the organic acid automatic supply unit, the organic acid is supplied to the fine particle formation unit by a certain amount. Thus, it is possible to reduce the time and effort for the user to charge the organic acid. In addition, the time until generation of fine particles is shortened, which contributes to power saving and energy saving. Further, deterioration of the organic acid due to repeated heating and cooling can be prevented.

Note that the exemplary embodiment described above is to exemplify the techniques in the present disclosure, and thus, various modifications, replacements, additions, omissions, and the like can be made in the scope of claims or in an equivalent scope of the claims.

### INDUSTRIAL APPLICABILITY

The present disclosure is applicable to a hairdressing appliance that imparts a beauty effect to the skin or hair. Specifically, the present disclosure is applicable to a hair dryer, a straight iron, a curl iron, a facial treatment device, a scalp care device, a nail care device, and the like.

### REFERENCE MARKS IN THE DRAWINGS

10: housing
12: cosmetic material holder
12a: jet unit
12b: lid
14: cosmetic material
15: cosmetic material
16: heating unit
18: controller
20: power supply
22: switch
24: tubular part
26: jet unit
26a, 26b: jet unit
28: fine particle
30: fan
32: motor
34: heater
36: partition wall
38: blower port
40: blower port
40A, 40B: iron part
42: brush
44: attachment
46: head
48: water tank
50: heating unit
52: jet unit
54: cylinder
56: carry-out unit
58: screw
60, 70: motor
72: heating unit
74: fan
76: laser light heater
78: motor
80: laser light source
82a, 82b, 82c: lens
84: galvanometer mirror
86: drive motor
92: rotary blade
100: cylindrical container
100A: hair dryer
100B: straight iron
100C: heat brush
100D: facial treatment device
100E: steamer
102: cosmetic material holder
104: filter
106: cosmetic material
108: pulverizing beads
110, 120, 126: fine particle
114, 116: roller
118: cosmetic material
122: ultrasonic vibrator
123: power supply
124: horn
130: housing
132: fan
134: motor
136: heater
138: temperature measurement unit
140: hot air controller
142: cosmetic material holder
144: cosmetic material
146: fine particle
148: frame
150: mesh
152: opening

## Claims

1. A cosmetic material comprising:
at least one organic substance, the at least one organic substance each being changeable to fine particles having an average particle size of less than or equal to 1 µm when being heated or pulverized,
wherein the cosmetic material is solid at normal temperature and does not contain a liquid component, a cream-like component, or a gel-like component.

2. The cosmetic material according to Claim 1, wherein each of the at least one organic substance is an organic acid.

3. The cosmetic material according to Claim 2, wherein the organic acid is at least one selected from the group consisting of a saturated fatty acid, a dicarboxylic acid, a hydroxycarboxylic acid, and an amino acid.

4. The cosmetic material according to Claim 1, wherein each of the at least one organic substance is a lipid or chitosan.

5. The cosmetic material according to any one of Claims 1 to 4, the cosmetic material comprising two or more of organic substances that are the at least one organic substance, wherein the organic substances have different melting points.

6. A hairdressing appliance comprising:
a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component;
a heating unit that heats the cosmetic material held by the cosmetic material holder; and
a jet unit that jets fine particles generated by heating the cosmetic material with the heating unit to an outside.

7. The hairdressing appliance according to Claim 6, the hairdressing appliance further comprising an air blower that sends out the fine particles generated by heating to the outside through the jet unit by blowing air.

8. The hairdressing appliance according to Claim 6 or 7, wherein the heating unit heats only a part of the cosmetic material within a certain period of time.

9. The hairdressing appliance according to any one of Claims 6 to 8, wherein
the cosmetic material held by the cosmetic material holder is in a powder form,
the hairdressing appliance further includes a cosmetic material conveyor that conveys the cosmetic material in a powder form from the cosmetic material holder toward the heating unit, and a conveyor controller that controls the cosmetic material conveyor, and
the conveyor controller performs control to obtain intermittent conveyance of the cosmetic material toward the heating unit.

10. The hairdressing appliance according to any one of Claims 6 to 8, wherein
the cosmetic material held by the cosmetic material holder is in a solid form,
the hairdressing appliance further includes a movement mechanism that relatively moves the cosmetic material in a solid form and the heating unit to switch the cosmetic material in a solid form and the heating unit to either a contact state or a non-contact state, and a movement mechanism controller that controls the movement mechanism, and
the movement mechanism controller performs control to obtain intermittent switching between the contact state and the non-contact state of the cosmetic material in a solid form and the heating unit.

11. The hairdressing appliance according to any one of Claims 6 to 8, wherein
the heating unit is a laser light heater that heats the cosmetic material by irradiation with laser light,
the hairdressing appliance further includes a laser light controller that controls at least one of on/off and output of the laser light of the laser light heater, and the cosmetic material holder holds, in addition to the cosmetic material, a heating auxiliary agent that generates heat through irradiation of the laser light of the laser light heater, and
the laser light controller performs control to obtain at least one of intermittent on/off of irradiation of the cosmetic material and the heating auxiliary agent held by the cosmetic material holder with the laser light and intermittent switching of a level of output of the laser light.

12. The hairdressing appliance according to any one of Claims 6 to 11, wherein
the cosmetic material holder includes two or more cosmetic material holders, and types of organic substances respectively held by the two or more cosmetic material holders are different from each other, and
the hairdressing appliance further includes a heating unit controller that recognizes the types of the organic substances respectively held in the two or more cosmetic material holder and controls the heating unit based on the types of the organic substances that are recognized.

13. A hairdressing appliance comprising:
a cosmetic material holder that holds a cosmetic material, the cosmetic material being solid at normal temperature, containing at least one organic substance, the at least one organic substance each being changeable to fine particles having an average particle size of less than or equal to 1 µm when being pulverized, and the cosmetic material not containing a liquid component, a cream-like component, or a gel-like component;
a pulverizer that pulverizes the cosmetic material held by the cosmetic material holder; and
a jet unit that jets fine particles generated by pulverizing the cosmetic material with the pulverizer to an outside.

14. A hairdressing appliance comprising:
a cosmetic material holder that holds a cosmetic material, the cosmetic material being solid at normal temperature, containing at least one organic substance, the at least one organic substance each being changeable to fine particles having an average particle size of less than or equal to 1 µm when being heated, and the cosmetic material not containing a liquid component, a cream-like component, or a gel-like component;
a heating unit that heats the cosmetic material held by the cosmetic material holder; and
a jet unit that jets fine particles generated by heating the cosmetic material with the heating unit to an outside,
wherein the heating unit heats the cosmetic material by supplying hot air to the cosmetic material holder.

15. The hairdressing appliance according to Claim 14, the hairdressing appliance further comprising a temperature measurement unit that measures a temperature of the hot air, and a hot air controller that controls at least one of the temperature of the hot air and a volume of the hot air based on the temperature measured by the temperature measurement unit.
